# EUROPÄISCHE PATENTSCHRIFT

(11) **EP 0 440 752 B1**
(45) Veröffentlichungstag und Bekanntmachung des Hinweises auf die Patenterteilung: **08.03.1995**
(21) Anmeldenummer: 90904244.2
(22) Anmeldetag: 08.03.1990
(51) Int. Cl.: C07J 11/00

(54) **VERFAHREN ZUR HERSTELLUNG VON 3-DESOXY-4-EN-STEROIDEN**
METHOD FOR THE PREPARATION OF 3-DESOXY-4-ENE STEROIDS
PROCEDE DE PRODUCTION DE 3-DESOXY-4-ENE-STERO DES

(30) Priorität: 21.03.1989 DE 3909770
(43) Veröffentlichungstag der Anmeldung: 14.08.1991
(73) Patentinhaber: SCHERING AKTIENGESELLSCHAFT, 13342 Berlin (DE)
(72) Erfinder: WINTERFELDT, Ekkehard, D-3004 Iserhagen 2 (DE); TILSTAMM, Ulf, D-3000 Hannover 1 (DE); HOFMEISTER, Helmut, D-1000 Berlin 28 (DE); LAURENT, Henry, D-1000 Berlin 28 (DE)
(86) Internationale Anmeldenummer: DE9000183
(87) Internationale Veröffentlichungsnummer: WO9011290

(56) Entgegenhaltungen:
- EP-A- 0 034 114
- DE-A- 2 158 837
- US-A- 3 471 531
- Chemical Abstracts, vol. 110, no.11, 13 March 1989, (Columbus, Ohio,US); Shinozaki Teizo: "Preparation of 2-(aminomethy) pyrrolodine", see page 683, abstract 94990n, & Tokkyo Koho JP 63,183,561, 28 July 1988

## Beschreibung

Die Erfindung betrifft ein Verfahren zur Herstellung von 3-Desoxy-4-en-Steroiden der allgemeinen Formel I 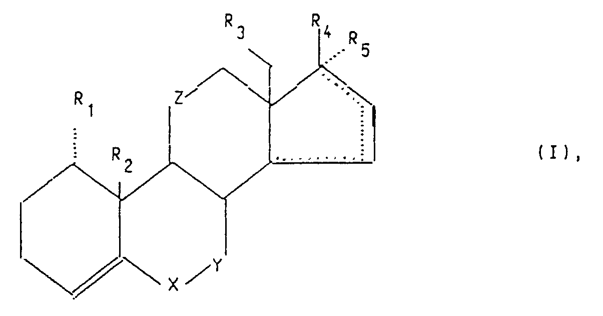
worin
- R₁, R₂ und R₃: ein Wasserstoffatom oder eine Methylgruppe bedeuten,
- R₄: eine niedere Alkylgruppe, eine Phenylgruppe oder eine freie, veresterte oder veretherte Hydroxygruppe darstellt,
- R₅: ein Wasserstoffatom, eine Vinylgruppe oder die Gruppierung -C≡CR₆ mit R₆ in der Bedeutung eines Wasserstoffatoms, einer Alkylgruppe mit maximal 4 Kohlenstoffatomen oder eines Halogenatoms symbolisiert,
- X: eine Methylengruppe, eine Fluormethylengruppe, eine Ethylidengruppe oder eine Vinylidengruppe darstellt,
- Y: eine Methylengruppe oder eine Ethylidengruppe darstellt und
- Z: eine Methylengruppe, eine Ethylidengruppe, eine Vinylidengruppe, eine Chlormethylengruppe oder eine Hydroxymethylengruppe symbolisiert und worin
die Bindungen ....., drei Einfachbindungen oder eine Doppelbindung und zwei Einfachbindungen oder eine konjugierte Doppelbindung darstellen, welches dadurch gekennzeichnet ist, daß man ein 3-Oxo-4-en-Steroid der allgemeinen Formel II 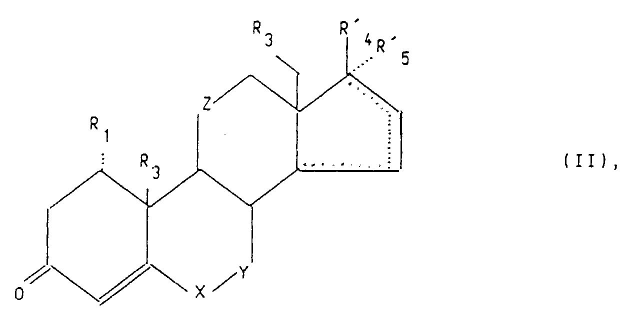
worin ....,
R₁, R₂, R₃, X, Y und Z die obengenannte Bedeutung besitzen und R'₄ und R'₅ das gleiche wie R₄ und R₅ bedeuten oder gemeinsam eine Oxogruppe darstellen, mit einer Reaktionsmischung aus Trifluoressigsäure oder Trichloressigsäure, gegebenenfalls einer weiteren Carbonsäure und Natriumborhydrid reduziert.

3-Desoxy-4-en-Steroide der allgemeinen Formel I sind bekanntlich pharmakologisch wirksame Substanzen oder wertvolle Zwischenprodukte zur Herstellung pharmakologisch wirksamer Verbindungen (DE-A 2 361 120; EP-B 17 094 und EP-B 34 114).

So zeichnen sich beispielsweise 3-Desoxy-4-en-Steroide der allgemeinen Formel Ia 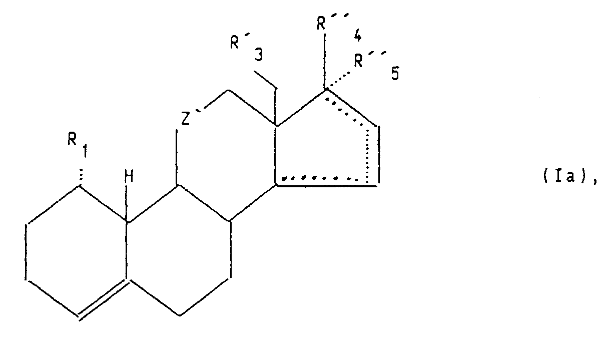
worin
- R'₃: ein Wasserstoffatom oder eine Methylgruppe
- R''₄: eine Hydroxygruppe oder Alkanoyloxygruppe mit bis zu 4 Kohlenstoffatomen,
- R''₅: ein Wasserstoffatom, eine Vinylgruppe, eine Ethinylgruppe, eine Chlorethinylgruppe oder eine 1-Propinylgruppe darstellen,
- X: eine Methylengruppe oder Vinylidengruppe symbolisiert und
- ....: eine Einfachbindung oder eine Doppelbindung bedeutet, durch eine starke gestagene Wirksamkeit aus. Beispiele derartig gestagen wirksamer Steroide sind das Lynestrenol (17α-Ethinyl-4-estren-17β-ol) und das Desogestrel (17α-Ethinyl-18-methyl-11-methylen-4-estren-17β-ol).

Nach dem bekannten Stand der Technik werden die 3-Desoxy-4-en-Steroide der allgemeinen Formel I aus den 3-Oxy-4-en-Steroiden der allgemeinen Formel II in der Weise hergestellt, daß man letztere beispielsweise mittels Ethandithiol in 3-Thioketale überführt und in letzteren mittels Natrium oder Lithium in flüssigem Ammoniak die Thioketalgruppe abspaltet (DE-A-2 158 837). Dieses Verfahren ist wegen der aufzuwendenden hohen Energiekosten recht aufwendig und hat darüberhinaus den Nachteil, daß es wegen der starken Geruchsbelästigung sehr umweltbelastend ist.

Demgegenüber ist das erfindungsgemäße Verfahren eine einfach durchzuführende, gering umweltbelastende einstufige Reaktion, bei welcher man ebenso wie bei dem vorbekannten zweistufigen Verfahren Ausbeuten von ca. 60 bis 85 % der Theorie erzielt.

Das erfindungsgemäße Verfahren wird zweckmäßiger Weise so durchgeführt, daß man primär das Natriumborhydrid bei einer Temperatur von -10° C bis +20° C in der Trifluoressigsäure oder Trichloressigsäure und gegebenenfalls einer weiteren Carbonsäure umsetzt. Da es hierbei infolge Wasserstoffentwicklung zu einer starken Schaumbildung kommt, setzt man den Trihalogenessigsäuren vor Beginn der Reaktion zweckmäßigerweise noch Acetonitril zu, wodurch die Schaumbildung weitgehend unterdrückt wird. Bei Verwendung von Trifluoressigsäure ist es erforderlich, bei Verwendung von Trichloressigsäure ist es nicht immer notwendig den der Trihalogenessigsäuren weitere Carbonsäuren zuzusetzen. Solche Carbonsäuren können Monocarbonsäuren, Dicarbonsäuren oder Tricarbonsäuren sein, wie zum Beispiel die Propionsäure, die Buttersäure, die Pivalinsäure, die Bernsteinsäure, die Zitronensäure oder insbesondere die sehr preisgünstige Essigsäure. In den nachfolgenden Ausführungsbeispielen werden zur Umsetzung des Natriumborhydrids mit den Trihalogenessigsäuren stets ein Überschuß an Reaktionsmittel/Losungsmittel-Gemisch verwendet, so daß sich klare Lösungen bilden. Die für eine technische Durchführung jeweils erforderliche minimale Menge an Reaktionsmittel/Lösungsmittel-Gemisch und dessen optimale Zusammensetzung muß im Einzelfall durch Vorversuche, wie sie dem Fachmann geläufig sind, ermittelt werden.

In die so dargestellte Natriumborhydrid/Trihalogenessigsaure-Reaktionsmischung, welche gegebenenfalls eine weitere Carbonsäure enthalt, kann man das umzusetzende Steroid - in einem inerten Lösungsmittel gelöst -eintragen. Reinerere Verfahrensprodukte erhalt man aber oft, wenn man diese Reaktionsmischung in eine Lösung des Steroids einträgt. Pro mol umzusetzendes Steroid verwendet man normalerweise aus 2,5 mol bis 20 mol Natriumborhydrid hergestellte Reaktionsmischung. Die Reaktionstemperatur betragt etwa -30° C bis +30° C. Die optimale Zugabegeschwindigkeit der einen Komponente zur anderen muß mittels der dem Fachmann geläufigen Vorversuche ermittelt werden; eine zu langsame Zugabegeschwindigkeit ist für den Reaktionsverlauf selbst meist nicht von Nachteil. Die Reaktionszeit beträgt in der Regel 5 Minuten bis 120 Minuten.

Es ist relativ unkritisch, in welchem inerten Losungsmittel das umzusetzende Steroid gelost wird. Geeignete inerte Losungsmittel sind beispielsweise Ether, wie Diethylether, Diisopropylether, Dioxan, Tetrahydrofuran oder 1,2-Dimethoxyethan, chlorierte Kohlenwasserstoffe wie Methylenchlorid, Chloroform, Tetrachlorkohlenstoff oder flüssige aromatische Kohlenwasserstoffe wie Benzol, Toluol oder Xylole.

Das umzusetzende 3-Oxo-4-en-Steroid der allgemeinen Formel II kann - wie bereits dargelegt wurde, isolierte oder konjugierte Doppelbindungen - in der 15-Position, in der 14- und/oder 16-Position, in der Vinylgruppe des Substituenten R₅ oder dem Vinylidenrest X und/oder Z haben und/oder im Substiutenten R₅ eine Dreifachbindung besitzen ohne daß diese Gruppierungen bei der Reaktion angegriffen werden. 3-Oxo-4-en-Steroide mit einer veretherten Hydroxygruppe R₄ (vorzugsweise Alkylether mit 1 bis 4 Kohlenstoffatomen wie der Methylether, der Ethylether oder der tert.-Butylether) werden nicht gespalten jedoch können bei 3-Oxo-4-en-Steroiden mit einer veresterten Hydroxygruppe R₄ (vorzugsweise Alkanoylester mit 1 bis 6 Kohlenstoffatomen wie das Acetat, Propionat, Pivalat oder Benzoylester) partiell umgeestert oder reduzierend gespalten werden. Bei 3-Oxo-4-en-Steroiden, die Halogenatome (vorzugsweise Fluor- oder Chloratome) tragen erfolgt während der Reaktion keine Halogenabspaltung. 3-Oxo-4-en-Steroide mit einer Oxogruppe in der 17-Position werden zu den entsprechenden 17β-Hydroxy-Steroiden reduziert, welche aber problemlos wieder zu den entsprechenden 17-Oxo-Steroiden aufoxidiert werden können, wie einem der nachfolgenden Ausführungsbeispiele dargelegt wird.

Die nachfolgenden Ausführungsbeispiele dienen zur näheren Erlauterung der Erfindung.

### Beispiel 1

Zu 2 ml Trifluoressigsäure, 2 ml Eisessig und 2 ml Acetonitril gibt man unter Eis/Wasserkühlung portionsweise 220 mg wasserfreies Natriumborhydrid. Anschließend tropft man 288 mg 17β-Hydroxy-4-androsten-3-on in 5 ml trockenem Methylenchlorid zu, rührt bei Raumtemperatur und versetzt nach 30 Minuten vorsichtig mit gesättigter Natriumhydrogencarbonat-Lösung. Man extrahiert mit Methylenchlorid, wascht mit Wasser neutral und trocknet über Magnesiumsulfat. Der Rückstand wird über eine Kieselgelsäule mittels Methylenchlorid/Methanol (99+1) chromatographiert und man erhalt 240 mg 4-Androsten-17β-ol vom Schmelzpunkt 146-149° C.

### Beispiel 2

Zu 5 ml Trichloressigsäure und 5 ml Acetonitril werden unter Eis/Wasserkühlung portionsweise 370 mg wasserefreies Natriumborhydrid gegeben. Anschließend werden 500 mg 17β-Hydroxy-4-androsten-3-on in 9 ml Methylenchlorid zugetropft. Es wird bei Raumtemperatur gerührt und nach 30 Minuten wie in Beispiel 1 beschrieben, aufgearbeitet. Es werden 400 mg 4-Androsten-17β-ol vom Schmelzpunkt 144-147° C erhalten.

### Beispiel 3

570 mg wasserfreies Natriumborhydrid werden unter Eis/Wasserkühlung portionsweise zu 5,5 ml Trifluoressigsäure, 5,5 ml Eisessig und 10 ml Acetonitril so zugegeben, daß die Innentemperatur +10° C nicht übersteigt. Nach Beendigung der Wasserstoffentwicklung tropft man die Losung bei Raumtemperatur zu 274 mg 17β-Hydroxy-4-estren-3-on in 5 ml Methylenchlorid, rührt 30 Minuten und versetzt vorsichtig mit gesättigter Natriumhydrogencarbonat-Losung. Anschließend extrahiert man mit Methylenchlorid, wascht mit Wasser und trocknet über Magnesiumsulfat. Nach chromatographischer Reinigung des Rohproduktes an Kieselgel mit Methylenchlorid/Methanol (99+1) erhalt man 210 mg 4-Estren-17β-ol vom Schmelzpunkt 96-99° C.

### Beispiel 4

800 mg wasserfreies Natriumborhydrid werden unter Eis/Wasserkühlung zu 7 ml Trichloressigsäure, 7 ml Pivalinsäure und 10 ml Acetonitril so zugegeben, daß die Innentemperatur +10° C nicht übersteigt. Die Natriumborhydrid-Losung wird bei Raumtemperatur zu 385 mg 17β-Hydroxy-4-estren-3-on in 8 ml Methylenchlorid getropft. Nach 20 Minuten versetzt man vorsichtig mit gesättigter Natriumhydrogencarbonat-Losung und arbeitet wie in Beispiel 3 beschrieben auf. Nach Chromatographieren des Rohproduktes an Kieselgel mit Methylenchlorid/Methanol erhalt man 290 mg 4-Estren-17β-ol vom Schmelzpunkt 95-98° C.

### Beispiel 5

Analog Beispiel 3 werden 300 mg 17β-Hydroxy-18-methyl-4-estren-3-on mit der aus Natriumborhydrid hergestellten Reaktionsmischung umgesetzt. Nach chromatographischer Reinigung des Rohproduktes an Kieselgel mit Hexan/Essigester werden 230 mg 18-Methyl-4-estren-17β-ol vom Schmelzpunkt 116-117° C erhalten.

### Beispiel 6

Analog Beispiel 3 werden 500 mg 17α-Ethinyl- 17β-hydroxy-4-estren-3-on mit der aus wasserfreiem Natriumborhydrid hergestellten Reaktionsmischung umgesetzt. Das Rohprodukt wird nach Behandlung mit Aktivkohle in Aceton aus Aceton umkristallisiert. Ausbeute 320 mg 17α-Ethinyl-4-estren-17β-ol vom Schmelzpunkt 160-162° C.

### Beispiel 7

Analog Beispiel 3 setzt man 350 mg 17α-Ethinyl-17β-hydroxy-18-methyl-4-estren-3-on mit der aus wasserfreiem Natriumborhydrid hergestellten Reaktionsmischung um. Nach Chromatographieren an Kieselgel mit Hexan/Essigester erhalt man 240 mg 17α-Ethinyl-18-methyl-4-estren-17β-ol vom Schmelzpunkt 54° C.

### Beispiel 8

Analog Beispiel 3 läßt man 280 mg 17α-Ethinyl-18-methyl-17β-hydroxy-4,15-estradien-3-on mit der aus Natriumborhydrid hergestellten Reaktionsmischung reagieren. Das Rohprodukt wird an Kieselgel mit Hexan/Essigester chromatographiert. Ausbeute 190 mg 17α-Ethinyl-18-methyl-4,15-estradien-17β-ol vom Schmelzpunkt 82° C.

### Beispiel 9

Analog Beispiel 3 werden 830 mg 17α-Ethinyl-17β-hydroxy-18-methyl-11-methylen-4-estren-3-on mit der aus wasserfreiem Natriumborhydrid hergestellten Reaktionsmischung umgesetzt. Nach Chromatographieren des Rohproduktes an Kieselgel mit Hexan/Essigester und Umkristallisieren aus Hexan werden 560 mg 17α-Ethinyl-18-methyl-11-methylen-4-estren-17β-ol vom Schmelzpunkt 110° C erhalten.

### Beispiel 10

Analog Beispiel 3 setzt man 680 mg 17-Phenyl-4,14,16-androstatrien-3-on mit der aus wasserfreiem Natriumborhydrid hergestellten Reaktionsmischung um. Nach Chromatographieren an Kieselgel mit Hexan/Essigester erhalt man 550 mg 17-Phenyl-4,14,16-androstratrien vom Schmelzpunkt 97° C.

### Beispiel 11

Analog Beispiel 3 werden 930 mg 18-Methyl-17-phenyl-4,14,16-estratrien-3-on (Schmelzpunkt 191° C, hergestellt analog 17-Phenyl-4,14,16-estratrien-3-on (Literatur: E. Winterfeldt et al., Tetrahedron Letter, 27, 5833 (1986)) mit der aus wasserfreiem Natriumborhydrid hergestellten Reaktionsmischung umgesetzt. Es werden nach Chromatographieren des Rohproduktes an Kieselgel mit Hexan/Essigester 730 mg 18-Methyl-17-phenyl-4,14,16-estratrien vom Schmelzpunkt 93,5° C erhalten.

### Beispiel 12

Analog Beispiel 3 setzt man 360 mg 17α-Chlorethinyl-17β-hydroxy-18-methyl-11-methylen-4-estren-3-on mit der aus wasserfreiem Natriumborhydrid hergestellten Reaktionsmischung um. Nach Chromatographieren des Rohproduktes an Kieselgel mit Hexan/Essigester erhalt man 230 mg 17α-Chlorethinyl-18-methyl-11-methylen-4-estren-17β-ol vom Schmelzpunkt 165° C.

### Beispiel 13

Analog Beispiel 3 läßt man 680 mg 18-Methyl-11-methylen-4-estren-3,17-dion mit der aus wasserfreiem Natriumborhydrid hergestellten Reaktionsmischung reagieren. Das erhaltene Rohprodukt (600 mg) wird in 10 ml Aceton mit 0,8 ml einer 8N Chromtrioxid/Schwefelsäure-Losung (Jones Reagenz) umgesetzt. Das Reaktionsgemisch gibt man in Eis/Wasser. Das ausgefallene Produkt wird abgesaugt, mit Wasser gewaschen und getrocknet. Nach Chromatographieren an Kieselgel mit Hexan/Essigester und nach Umkristallisieren aus Diethylether erhalt man 340 mg 18-Methyl-11-methylen-4-estren-17-on vom Schmelzpunkt 98° C.

## Patentansprüche

1. Verfahren zur Herstellung von 3-Desoxy-4-en-Steroiden der allgemeinen Formel I 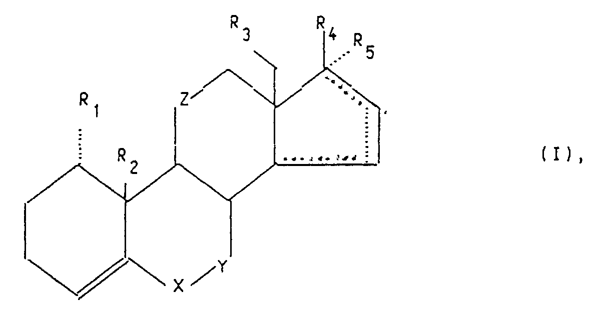 worin
R₁, R₂ und R₃ ein Wasserstoffatom oder eine Methylgruppe bedeuten,
R₄ eine niedere Alkylgruppe, eine Phenylgruppe oder eine freie, veresterte oder veretherte Hydroxygruppe darstellt,
R₅ ein Wasserstoffatom, eine Vinylgruppe oder die Gruppierung -C≡CR₆ mit R₆ in der Bedeutung eines Wasserstoffatoms, einer Alkylgruppe mit maximal 4 Kohlenstoffatomen oder eines Halogenatoms symbolisiert,
X eine Methylengruppe, eine Fluormethylengruppe, eine Ethylidengruppe oder eine Vinylidengruppe darstellt,
Y eine Methylengruppe oder eine Ethylidengruppe darstellt und
Z eine Methylengruppe, eine Ethylidengruppe, eine Vinylidengruppe, eine Chlormethylengruppe oder eine Hydroxymethylengruppe symbolisiert und worin
die Bindungen ....., drei Einfachbindungen oder eine Doppelbindung und zwei Einfachbindungen oder eine konjugierte Doppelbindung darstellen, dadurch gekennzeichnet, daß man ein 3-Oxo-4-en-Steroid der allgemeinen Formel II 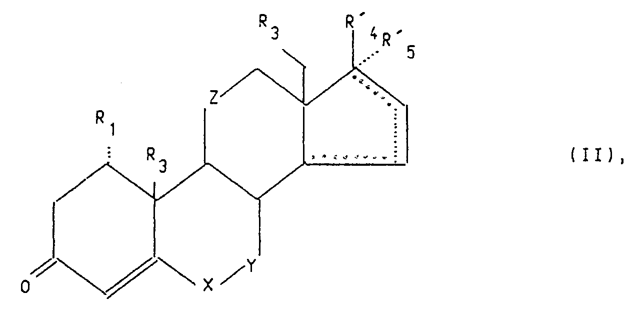 worin .....,
R₁, R₂, R₃, X, Y und Z die obengenannte Bedeutung besitzen und R'₄ und R'₅ das gleiche wie R₄ und R₅ bedeuten oder gemeinsam eine Oxogruppe darstellen, mit einer Reaktionsmischung aus Trifluoressigsäure oder Trichloressigsäure, gegebenenfalls einer weiteren Carbonsäure und Natriumborhydrid reduziert.

## Claims

1. A process for the preparation of 3-deoxy-4-ene steroids of the general formula I 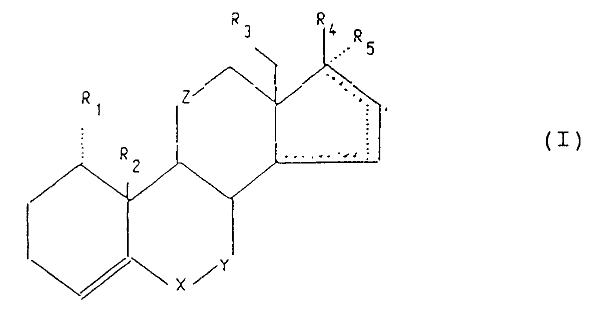 wherein
R₁, R₂ and R₃ are a hydrogen atom or a methyl group,
R₄ is a lower alkyl group, a phenyl group or a free, esterified or etherified hydroxy group,
R₅ is a hydrogen atom, a vinyl group or is the grouping -C≡CR₆ wherein R₆ is a hydrogen atom, an alkyl group having a maximum of 4 carbon atoms or a halogen atom,
X is a methylene group, a fluoromethylene group, an ethylidene group or a vinylidene group,
Y is a methylene group or an ethylidene group, and
Z is a methylene group, an ethylidene group, a vinylidene group, a chloromethylene group or a hydroxymethylene group,
and wherein
the bonds ..... are three single bonds, or a double bond
and two single bonds, or a conjugated double bond, characterised in that a 3-oxo-4-ene steroid of the general formula II 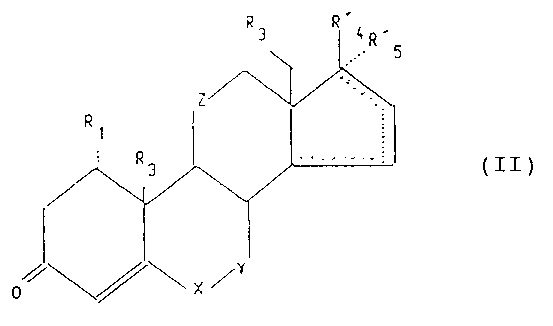 wherein
....., R₁, R₂, R₃, X, Y and Z have the meanings given above and
R'₄ and R'₅ have the same meanings as R₄ and R₅ or together form an oxo group,
is reduced with a reaction mixture comprising trifluoroacetic acid or trichloroacetic acid, optionally a further carboxylic acid, and sodium borohydride.

## Revendications

1. Procédé pour la préparation de 3-désoxy-4-èn-stéroïdes de formule générale I 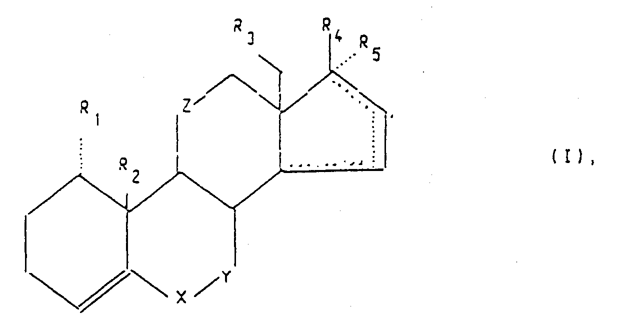 où
R₁, R₂ et R₃ représentent un atome d'hydrogène ou un groupe méthyle,
R₄ représente un groupe alkyle inférieur, un groupe phényle ou un groupe hydroxy libre, estérifié ou éthérifié,
R₅ représente un atome d'hydrogène, un groupe vinyle ou le groupement -C≡CR₆ avec R₆ ayant la signification d'un atome d'hydrogène, un groupe alkyle avec au maximum 4 atomes de carbone ou un atome d'halogène,
X représente un groupe méthylène, un groupe fluorométhylène, un groupe éthylidène ou un groupe vinylidène,
Y représente un groupe méthylène ou un groupe éthylidène, et
Z représente un groupe méthylène, un groupe éthylidène, un groupe vinylidène, un groupe chlorométhylène ou un groupe hydroxyméthylène et
où les liaisons ....., représentent trois liaisons simples ou une liaison double et deux liaisons simples ou une liaison double conjuguée, lequel procédé est caractérisé en ce qu'on réduit un 3-oxo-4-èn-stéroïde de formule générale II 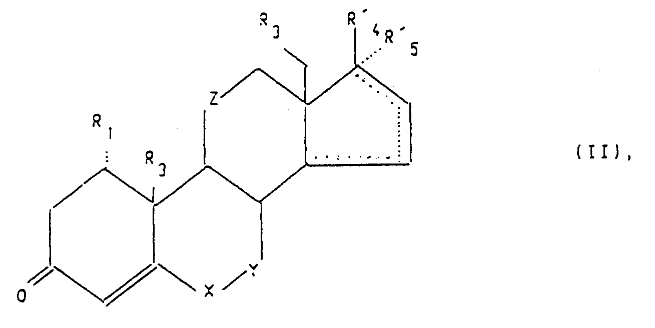 où .....
R₁, R₂, R₃, X, Y et Z ont la signification donnée ci-dessus et R'₄ et R'₅ ont la même signification que R₄ et R_{5,} ou ensemble représentent un groupe oxo, par un mélange réactionnel composé d'acide trifluoracétique ou trichloracétique, éventuellement d'un autre acide carboxylique, et de borohydrure de sodium.
